# EUROPEAN PATENT SPECIFICATION

(11) **EP 3 030 516 B1**
(45) Date of publication and mention of the grant of the patent: **07.06.2017**
(21) Application number: 14834890.7
(22) Date of filing: 06.08.2014
(51) Int. Cl.: B82Y 5/00, B82Y 15/00, A61K 38/48, G01N 33/50, C08G 85/00, A61K 49/00, A61P 35/00, A61K 9/16, A61K 31/337, A61K 31/4745, A61K 31/513, A61K 31/555, A61K 33/24, B82Y 40/00, A61K 47/52

(54) **THE SYNTHESIS OF CORE-SHELL METAL-SEMICONDUCTOR NANOMATERIALS**
SYNTHESE VON KERN-HÜLLEN-METALLHALBLEITER-NANOMATERIALIEN
SYNTHÈSE DE NANOMATÉRIAUX MÉTAL SEMICONDUCTEUR DE TYPE COEUR-ÉCORCE

(30) Priority: 07.08.2013 ZA 201305925
(43) Date of publication of application: 15.06.2016
(73) Proprietor: University of Zululand, 3886 KwaDlangezwa (ZA)
(72) Inventor: REVAPRASADU, Neerish, 3886 KwaDlangezwa (ZA); DUNPALL, Rekha, 3886 KwaDlangezwa (ZA)
(74) Representative: Awapatent AB
(86) International application number: PCT/IB2014/063729
(87) International publication number: WO 2015/019297

(56) References cited:
- WO-A1-2006/118542
- WO-A1-2006/118542
- WO-A2-2009/111470
- US-A1- 2008 290 936
- US-B2- 6 649 138
- US-B2- 8 198 336
- RAJASEKHAR PULLABHOTLA V S R ET AL: "A novel route to cysteine capped Au-CdSe hybrid nanoparticles", MATERIALS LETTERS, NORTH HOLLAND PUBLISHING COMPANY. AMSTERDAM, NL, vol. 63, no. 24-25, 15 October 2009 (2009-10-15), pages 2097-2099, XP026467341, ISSN: 0167-577X, DOI: 10.1016/J.MATLET.2009.06.057 [retrieved on 2009-07-04]
- NHLAKANIPHO MNTUNGWA ET AL: "A facile hybrid route to luminescent ZnTe nanoparticles", MATERIALS LETTERS, NORTH HOLLAND PUBLISHING COMPANY. AMSTERDAM, NL, vol. 81, 26 April 2012 (2012-04-26), pages 108-111, XP028491537, ISSN: 0167-577X, DOI: 10.1016/J.MATLET.2012.04.131 [retrieved on 2012-05-04]
- ZHENHUA SUN ET AL: "A General Approach to the Synthesis of Gold-Metal Sulfide Core-Shell and Heterostructures", ANGEWANDTE CHEMIE INTERNATIONAL EDITION, vol. 48, no. 16, 13 March 2009 (2009-03-13), pages 2881-2885, XP055287833, DE ISSN: 1433-7851, DOI: 10.1002/anie.200806082
- HUSSAIN, A. M. P. ET AL.: 'Size quantization effect in highly stable UV emitting HgTe nanoparticles: Structure and optical properties' JOURNAL OF APPLIED PHYSICS vol. 106, 2009, pages 094306-1 - 094306-5, XP012127832
- LAW, W. C. ET AL.: 'Aqueous-Phase Synthesis of Highly Luminescent CdTe/ZnTe Core/Shell Quantum Dots Optimized for Targeted Bioimaging' SMALL vol. 5, no. 11, 2009, pages 1302 - 1310, XP055259937
- DUNPALL, R. ET AL.: 'An In Vitro Assessment of the Interaction of Cadmium Selenide Quantum Dots with DNA, Iron, and Blood Platelets' IUBMB LIFE vol. 64, no. 12, 2012, pages 995 - 1002, XP055259926

## Description

### INTRODUCTION

This invention relates to a method for the synthesis of core/shell, semiconductor nanomaterials and more particularly, the synthesis of gold-zinc telluride core/shell nanoparticles that are suitable for use, inter alia, as drug carriers.

### BACKGROUND OF THE INVENTION

During the last decade, substantial effort has been made with the preparation of wide-band-gap II-IV (group 12 to 16) nanometer scale semiconductors in the light of their electronic and optical properties. A method for producing such semiconductors, in the form of ZnTe - Semiconductor Nanorods, was described by Y. Li in Advanced Materials 1999, 11, No 10, 847 - 850. The method comprised of a solvothermal process and a subsequent thermal treatment using Zn and Te as the reagents with hydrazine (N₂H₄·H₂O) as the solvent.

Another nanoparticle is described in European Patent Application EP 2 405 037 A1, with a priority date of 12 September 2005. This nanoparticle comprised of a core semiconductor material, a first layer, comprising a first semiconductor material provided on the core, and a second layer, comprising a second semiconductor material provided on the first layer, the core semiconductor material being different to the first semiconductor material and the first semiconductor material being different to the second semiconductor material. The method included effecting conversion of a nanoparticle core precursor composition to the material of the nanoparticle core, depositing the first layer on the core and then depositing the second layer on the first layer, the core precursor composition comprising a first precursor species containing a first ion to be incorporated into the growing nanoparticle core and a separate second precursor species containing a second ion to be incorporated into the growing nanoparticle core, the conversion being effected in the presence of a molecular cluster compound under conditions permitting seeding and growth or the nanoparticle core.

A method for preparing core/shell structure nanoparticles is described in United States Patent 8,017,181 B2, with a priority date of 2 May 2006. This method endeavoured to provide a rapid and reproducible process for preparing a core/shell structure nanoparticle that is less sensitive to the fluctuations of reaction conditions when compared to conventional so-called drop wise methods. This method for preparing core/shell nanoparticles comprised firstly, the step of dissolving a shell precursor in a solvent to form a shell precursor solution, and then allowing the shell precursor solution to be stabilized at a temperature sufficiently high for the shell precursor to grow an overcoat on the surface of a core nanoparticle and, secondly, the step of adding a powder form of the core nanoparticle into the stabilized shell precursor solution.

Other core/shell nanoparticles and methods for preparing such particles are described in United States Patent Application 2010/0283005 A1, with a priority date of 28 September 2007. These nanoparticles comprise a core that that itself comprises a core of a first material and a layer comprising a second material, wherein one of the first and the second materials is a semiconductor material, incorporating ions from group 13 and group 15 of the periodic table and the other of the first and the second materials is a metal oxide material, incorporating metal ions selected from any one of the groups 1 to 12, 14 and 15, respectively of the periodic table.

Capping agents for semiconductor nanoparticles are described in United States Patent Application 2009/0212258 A1, with a priority date of 25 February 2008.

United States Patent Application 2009/0212258 A1 shows that the size of a semiconductor nanoparticle helps determine the electronic properties of the material; that the band-gap energy may be inversely proportional to the size of the semiconductor nanoparticle as a consequence of quantum confinement effects; and that the large surface area to volume ratio of the nanoparticle affects the physical and chemical properties of the nanoparticle.

The prior art further shows that single core nanoparticles that include a single semiconductor material typically have relatively low quantum efficiencies. These low quantum efficiencies apparently arise from nano-radiative electron-hole recombinations that occur at defects and dangling bonds at the surface of the nanoparticle. The prior art further shows that core/shell nanoparticles typically include a single semiconductor core material that has a shell of a second semiconductor material grown epitaxially on the surface of the core. The shell material usually has a winder band-gap and similar lattice dimensions to the core semiconductor material. The intention of adding the shell may be to eliminate defects and dangling bonds from the surface of the core, and thereby charge carriers within the core and away from surface states that may function as centers for non-radiative recombination.

Yet, the surfaces of core, core/shell, and core-multi-shell nanoparticles may have highly reactive dangling bonds. These may be passivated by capping the surface atoms with organic ligand molecules that inhibit aggregation of particles, protect the particle from its surrounding chemical environment, and (at least in the case of core nanoparticles) provide electronic stabilization. The capping ligand compound may be the solvent that is employed in the core growth and/or shelling of the nanoparticles. Either way, the ligand compound caps the surface of the nanoparticle by donating lone-pair electrons to the surface metal atoms of the nanoparticle.

Nanoparticles may typically be synthesized in the presence of a lipophilic ligand compound, resulting in nanoparticles that may be soluble in non-polar media. To decrease or eliminate this solubility, the ligand compound may be exchanged for a different ligand compound of greater polarity; however, the quantum yield of the nanoparticles diminishes as a result.

The resulting semiconductor nanoparticles may be used in a range of different applications, in which the nanoparticles may be externally excited by photoexcitation, electro-excitation, or another form of excitation, leading to electron-hole recombination and subsequent emission of photons in the form of light of a predetermined wavelength, e.g. visible light. The use of surface functionalized nanoparticles in such applications has so far, however, been limited by the loss in quantum yield upon surface functionalization.

The compounds and methods disclosed in United States Patent Application 2009/0212258 A1 are thus aimed at overcoming the problems with methods existing at that time for the surface functionalization of semiconductor nanoparticles which have previously hindered the use of surface functionalized nanoparticles in such applications.

Nanoparticles with increased functionality, such as bi-functionality and multi-functionality, comprising nanoparticles of different compositions fused together forming heterostructures of interlinked nanoparticles, are described in PCT patent Application 2008/02388. The aim of that application is to provide such nanoparticle materials and with more robust and/or enhanced optical properties. These nanoparticle comprise a core, comprised of a first material and layer, comprised of a second material, wherein one of the first and second materials is a semiconductor material incorporating ions from group 13 and group 15 of the periodic table and the other of the first and second materials is a metal oxide material incorporating metal ions selected from any one of groups 1 to 12, 14 and 15 of the periodic table.

The use of gold nanoparticles specifically, and particularly in biological applications such as for labelling, delivering, heating and sensing, together with the underlying mechanisms and the specific features of the gold nanoparticles required for such applications, are described in Chemical Society Review 2008, 37, 1896 - 1908. Even though the use of colloidal gold nanoparticles, due to their optical properties, can be dated back to ancient times for applications such as glass staining, and the investigations on gold colloids can be dated to Faraday, the use of gold nanoparticles can only be dated back to the late 1990's for biological applications, i.e. the discovery of bio-nanotechnology. More particularly, as modern technology enables controlled synthesis and functionalization of nanomaterials materials, investigation into the particular properties of colloidal gold and the exploration of novel techniques.

The prior art substantially discloses the synthesis of gold nanoparticles in a relatively wide range of nanometres in both aqueous solutions and in organic solvents. Typically, gold salts such as AuCl₃ are reduced by the addition of a reducing agent which leads to the nucleation of Au ions to nanoparticles. Correspondingly, stabilizing agents are required and are either adsorbed or chemically bound to the surface of the Au nanoparticles. These stabilizing agents or so-called surfactants are typically charged so as to enable the equally charged nanoparticles to repel each other so as to stabilise the particles colloidally.

The prior art shows the synthesis of gold nanoparticles in aqueous solutions commonly conducted with citric acid serving first to reduce the gold salt and trigger nucleation, and secondly to stabilise the particle colloidally by its negative charges as its adsorbs to the particles. The prior art also shows the synthesis of gold nanoparticles in organic solvents, using different agents for reducing and for stabilizing the gold salt. Where particles are frequently dispersed in organic solvents, stabilising agents/surfactants, such as those based on hydrophobic alkane chains, are bound to the gold particle surface in order to provide colloidal stability. The prior art further shows the growing of gold particles comprising of spherical, rod and hollow shells like geometries.

Colloidal gold nanoparticles are typically surrounded by a shell of stabilizing molecules. With one of their ends, these molecules are either adsorbed or chemically linked to the gold surfaces, while the other ends point towards the solutions and provide colloidal stability. After synthesis of the gold nanoparticles, the stabilizer molecules can be replaced by other stabilizer molecules in a ligand exchange reaction. As thiol moieties bind with high affinity to gold surfaces, most frequently thiol-modified ligands are used which bind to the surface of the Au particles (which are by several groups also called "monolayer-protected clusters") by formation of Au-sulfur bonds. Ligand exchange is motivated by several aspects. Ligand exchange allows, for example, the transfer of Au particles from an aqueous to an organic phase (and vice versa) by exchange hydrophilic surfactants with hydrophobic surfactants (and vice versa). In this way, by choosing the surfactant molecules, it is possible to adjust the surface properties of the particles.

For applications in aqueous solution, thiol-based surfactants with carboxylic groups at the other end pointing towards to the solutions are typically used. These molecules provide colloidal stability due to their negative charges; in addition they can also be used as anchor points for the further attachment of biological molecules. Often poly(ethylene glycol) (PEG) is used as a ligand as PEG reduces nonspecific adsorption of molecules to the particle surface and it provides colloidal stability because particles with PEG brushes on their surface repel each other for steric reasons. Other surface coating techniques such as embedding particles in a silica shell have been used by several groups.

The prior art further shows that biological molecules can be attached to gold nanoparticles in several ways. For example, if the biological molecules have a functional group which can bind to the gold surface, such as thiols or specific peptide sequences, the biological molecules can replace some of the original stabilizer molecules when they are added directly to the particle solution. Accordingly, molecules such as oligonucleotides, peptides or PEG can be linked readily to gold nanoparticles and subsequent sorting techniques even allow such gold particles with an exactly defined number of attached molecules per particle to be obtained. Alternatively, biological molecules can also be attached to the shell of stabilizer molecules around the gold nanoparticles by bioconjugate chemistry. The most common protocol is the linkage of amino-groups on the biological molecules with carboxy groups at the free ends of stabilizer molecules by using EDC (1-ethyl-3-(3-dimethylaminopropyl)-carbodimide-HCl). With corresponding strategies, almost all kinds of biological molecules can be attached to the gold nanoparticle surface.

However, the prior art shows that even though the above processes are relatively well established, bioconjugation of gold nanoparticles is still not a simple exercise, and characterization of synthesized conjugates is necessary, in particular to rule out aggregation effects or unspecific binding during the conjugation reaction. More specifically, in many conjugation protocols, the number of attached molecules per gold nanoparticle is only a rough estimate, as no standard method for determining the surface coverage of particles modified with molecules has yet been established.

A novel method for producing aqueous compatible nanoparticles is described in United States Patent Application 2010/0059721 A1, with a priority date of 21 July 2008. The method is aimed at obviating or mitigating some of the challenges with in the then prior art methods for producing aqueous compatible nanoparticles. The disclosed method used a nanoparticles binding ligand, incorporating a nanoparticle binding group and a solubilising group precursor. The solubilising group precursor is converted to a solubiliser incorporating the solubilising group so as to affect binding of the binding ligand to the nanoparticles and thereby produce the aqueous compatible nanoparticles. The conversion of the solubilising group precursor can be effected by several methods, e.g., by contacting the nanoparticle binding ligand with a sufficient amount of a precursor modifying agent to convert substantially all of the solubilising group precursors present in the binding ligand to solubilising groups.

The usefulness in a variety of applications of combining multiple discrete components into single multi-functional nanoparticles is described in DOI:10.1038/NNANO.2009.193. This prior art shows that retention of the unique optical and electrical properties of each component after nanoscale integration has been problematic, particularly when trying to combine fiuorophores such as semiconductor nanoparticles or so-called quantum dots with plasmonic materials such as gold at a nano level, because gold and other metals can quench the fluorescence. So far, the combination of quantum dot fluorescence with plasmonically active gold has only been demonstrated on flat surfaces. The authors combined fluorescent and plasmonic activities in a single, nanoparticle by controlling the spacing between a nanoparticle or quantum dot core and an ultrathin gold shell with nanometre precision through layer-by-layer assembly. The wet-chemistry approach provides a general route for the depositing of ultrathin gold layers onto virtually any discrete nanostructure or continuous surface, and should prove useful for multi-modal bioimaging, interfacing with biological systems, reducing nanotoxicity, modulating electromagnetic fields and contacting nanostructures.

A so-called general approach to the synthesis of gold-metal sulphide core/shell and heterostructures is described in Angewandte DOI: 10:1002/anie.200806082. The method particularly relates to the synthesis of water dispersible gold-metal sulphide core/shell and heterostructures. Hybrid nanostructures made up of Au nanocrystals of varying shapes and different transition metal sulphide semiconductors (ZnS, CdS, Ag₂S, NiS, and CuS) have been successfully synthesized following a similar procedure.

This prior art shows that methods previously developed for the synthesis of metal-semiconductor nanostructures include selective growth of metals onto the tips and surfaces of semiconductor nanocrystals, photodeposition of metals on semiconductor nanorods, diffusion of metals into semiconductors, and growth of semiconductors on metal seeds. These methods often involve growth in organic solvents, and therefore yield nanostructures that are not suitable for photocatalytic cleaning and biological applications. In addition, most of the products obtained from these methods are heterostructures. Only a few examples of metal-semiconductor core/shell nanostructures have been reported prior to this publication, although the core/shell geometry can maximize the interfacial area and thus provide a platform for studying the plasmon-exciton interactions and charge distributions. Furthermore, with previous methods, control of the shapes and sizes of noble metal components remains a challenge. This controllability is highly important for studying the plasmon-exciton interactions and even tailoring them for improved application performances, because the plasmonic properties of noble metals are strongly dependent, on their shapes and sizes.

In addition and in Nano Today (2010) 5, 213 - 230, the functionalisation of nanoparticles with cores of inorganic materials such as noble, magnetic metals, their alloys and oxides, and semiconductors, as well as their potential for application in areas of biomedicine, from diagnostics to treatment of diseases, as described.

The publication recognised that the effects of nanoparticles must be predictable and controllable, and deliver the desired result with minimum cytotoxicity. The authors recognised that these criteria can be met by careful tailoring of the ligand shell, allowing stabilisation, specific targeting and recognition of biochemical species. For these reasons, the publication focused on the synthesis and biofunctionalisation of inorganic metal, semiconductor and magnetic nanoparticles for biomedical applications.

The applicant reported a route to cysteine capped Au-CdSe hybrid nanoparticles in Materials Letters 63 (2009) 2097 - 2099. In that publication, the synthesis of multicomponent nanostructured materials, the combining of two nanomaterials to provide a model structure for further understanding the properties of the heterostructures, the fact that the combination of a metal and a semiconductor as one material enables the properties of both parent materials to be harnessed in potential biomedical applications, and the fact that the metal can provide the anchor point for electrical connections and self assembly, are discussed. Most of the early studies of metal-semiconductor structures dwelled on the core/shell structure of metal/oxide by depositing oxide shells such as TiO₂ SnO₂, and SiO₂ on metal particles of Ag and Au.

The applicant pointed out in the above publication that Banin *et al.* has done extensive work on the growth of gold tips on anisotropic CdSe particles. By varying the concentration of gold they observed a transition from two sided growth to one sided gold growth. The growth of gold particles has been extended to other semiconductors such as CdS, InAs and ZnO.

The synthesis of these hybrid structures either in the form of alloys or core/shell systems involves two steps. Initially the semiconductor particles are synthesized at high temperatures in conventional coordinating solvents such TOPO. The gold particles are then grown in a separate procedure onto the semiconductor. An alternative to this procedure was described by Wang *et al.* who synthesized core/shell nanoparticles of Au/CdSe using a Au-Cd bi-alloy precursor. The Cd-Au precursor and tri-octylphosphine selenide (TOPSe) was thermolysed in a mixture of hexadecylamine (HDA) and trioctylphosphine oxide (TOPO) at high temperatures. The optical properties of the resultant crystalline material were intermediate between that of the Au core and CdSe shell.

The applicant further reported elsewhere the synthesis of highly water-soluble CdSe nanoparticles passivated with cysteine without the use of any additional stabilizer. The cysteine amino acid was chosen to cap the CdSe nanoparticles as it passivates the surface states of nanoclusters more effectively than other thiols thereby enhancing their emission properties. These bio-conjugated nanoparticles could be of utility as tags for protein, or DNA, recognition of specific antibodies or antigens through luminescence emission measurements and the potential development of biosensors. In this work the applicant adapted the synthetic method to synthesize Au-CdSe hybrid core/shell particles. Very briefly, a solution of HAuCl₄, Se and NaBH₄ was simultaneously mixed in a three necked flask under nitrogen. Under careful pH control, cysteine was added to the reaction mixture after a few minutes. A solution of CdCl₂ was then added after 3-4 h, and stirring continued overnight under an inert atmosphere. The resultant Au-CdSe particles were centrifuged, separated and dissolved in water or acetone for further analysis.

The Applicant also described the synthesis of semiconductor nanoparticles such as cadmium chalcogenides in Materials Letters 81 (2012) 108-111. The synthesis is a major focus of research in nanomaterials synthesis. However, the toxicity of cadmium has been a drawback which has compelled researchers to look at less toxic materials such as zinc chalcogenides. Among the zinc chalcogenides, ZnTe, is an attractive semiconductor for various applications in optoelectronic and thermoelectric devices. However, when compared with the sulphur and selenide zinc analogues there are fewer reports on the preparation of ZnTe nanocrystals. Some routes for the synthesis of ZnTe nanoparticles include the solvothermal process, pulsed laser ablation, mechanical milling and microwave plasma routes.

The applicant pointed out that Jun *et al.* reported the use of a monomeric molecular precursor, [Zn(TePh)₂][TMEDA] for the 'one pot' synthesis of dodecylamine capped ZnTe nanoparticles. The size and shape of the nanoparticles were controlled by temperature and the capping ligands. Recently the synthesis of wurtzite nanorods with controllable aspect ratios was reported. The dimensions of the particles were controlled by adopting active polytellurides as the tellurium precursor in conjunction with variation of the precursor concentration, reaction temperature and/or reaction time. Here the applicant reported the synthesis of HDA capped ZnTe nanoparticles by a hybrid method employing both solution chemistry and thermolysis at high temperatures. The work is a follow up on previous work on CdTe and PbTe. The facile scheme is reproducible and can be readily scaled up for industrial production.

The use of pre-grown Au nanoparticles or crystals for the synthesis of gold-metal sulphide nanostructures allows their shapes and sizes, and thus their plasmonic properties, to be rationally chosen.

The National Cancer Registry (NCR) and various international cancer research institutes provide statistics that show an increase in the prevalence of cancer patients worldwide. The fight against cancer has evolved into a multidisciplinary approach involving clinicians, chemists, biomedical engineers and materials scientists. Chemotherapy is a commonly used treatment for cancer. It involves the use of various cytotoxic drugs that often affect both cancer and healthy cells. This treatment often results in adverse side effects such as hair and weight loss, nausea, diarrhoea, cancer cachexia, opportunistic infections and sometimes even death. The challenge that researchers are faced with is the design of chemotherapeutic drugs that are not only cancer specific but also exhibit higher therapeutic efficacy. Advanced nanosystems such as core/shell nanoparticles have the potential to support this application if designed appropriately. The synthesis of heterostructured nanoparticles has received much attention recently due to their potential in catalysis, electronics and biomedical sciences. The combination of two different types of material that function as a single entity can confer particular advantages such as enhanced optical, electrical or structural properties. A challenge in designing such materials arises from determining the structure of a two-component system and in identifying compatible core/shell component elements. The controlled synthesis of bimetallic heterosystems such as Pd/Pt or Ag/Au core/shell systems is possible because of the relatively close match between the crystal lattices of their component parts, whereas core/shell metal-semiconductor systems are more difficult to synthesize their crystal structures differ due to the large crystal lattice mismatches. There have been few reports of metal-semiconductor core/shell nanostructures. As described above, Wang and co-workers described a general method for the preparation of Au nanocrystals of different shapes and sizes as starting material for the synthesis of water-dispersible Au-metal sulfide core/shell structures. Salant et al. conducted elaborate work on the growth of gold tips on anisotropic CdSe particles which was used to establish a relationship between the growth pattern and morphology that develops from a stabilized core. The synthesis of gold shelled heterostructures with the core composed of a magnetic nanomaterial such as iron oxide is more common.

There is however little regarding the combination of metal-semiconductor hybrid systems and their application in drug delivery for medical procedures such as tumour treatment.

### SUMMARY OF THE INVENTION

According to a first aspect of the invention there is provided a novel method for the synthesis of a metal-semiconductor nanomaterial, the method comprising the addition of reduced metal salt to a solution of prepared cysteine capped semiconductor telluride quantum dots, producing a solution of cysteine capped metal - semiconductor telluride core/shell nanoparticles.

According to a second aspect of the invention there is provided a novel method for the synthesis of metal-semiconductor nanomaterials, the method comprising the steps of:
Reducing a metal salt;
Providing a cysteine capped semiconductor telluride quantum dot solution; and
Adding the reduced metal salt to the solution of prepared cysteine capped semiconductor telluride quantum dots;
producing a solution of cysteine capped metal-semiconductor telluride core/shell nanoparticles.

According to a third aspect of the invention there is provided a metal-semiconductor nanomaterial, comprising of cysteine capped, metal-semiconductor telluride core/shell nanoparticles.

According to a fourth aspect of the invention there is provided a nano-scale drug delivery system comprising a cysteine capped metal-semiconductor telluride core/shell nanoparticle.

The metal may be gold, and the semiconductor may be zinc telluride.

According to a fifth aspect of the invention there is provided a novel method for the synthesis of metal-semiconductor nanomaterials, the method comprising the addition of a reduced gold salt to a solution of prepared cysteine capped zinc telluride quantum dots, producing a solution of cysteine capped gold-zinc telluride core/shell nanoparticles.

According to a sixth aspect of the invention there is provided a novel method for the synthesis of a metal-semiconductor nanomaterial, the method comprising the steps of:
Reducing a gold salt;
Adding reduced tellurium, zinc salt and cysteine to form a cysteine capped zinc telluride quantum dot solution; and
Adding the reduced gold salt to the solution of prepared cysteine capped zinc telluride quantum dots;
producing a solution of cysteine capped gold-zinc telluride core/shell nanoparticles.

According to a seventh aspect of the invention there is provided a metal-semiconductor nanomaterial, comprising of cysteine capped, gold-zinc telluride core/shell nanoparticles, characterised in having a gold core and a zinc telluride shell.

According to an eighth aspect of the invention there is provided a nano-scale drug delivery system comprising a cysteine capped gold-zinc telluride core/shell nanoparticle.

The drug delivery system may be characterised in that at least one active ingredient of a medicament is attached to the surface of the core/shell structure, preferably with a tumour specific receptor, for targeted drug delivery to such tumours.

The nanoparticles are preferable of between 4 and 40 nanometre diameters, preferably between 5 and 30 nanometres, and in one embodiment, the nanaoparticles have a diameter of approximately 30 nanometres.

The nanoparticles may be highly stable, water soluble cysteine capped, gold-zinc telluride core/shell nanoparticles.

The nanoparticles preferably have enhanced optical properties characterised by Ultraviolet-visible spectroscopy (UV/Vis) at 538 nm and Photoluminscence (PL) at a range of 400 to 420 nm and, preferably, when subjected to X-ray diffraction, both Au and ZnTe show the characteristic peaks.

The shell is preferably comprised of cubic zinc blended ZnTe. The core preferably comprises cubic Au.

The invention further provides a nanoparticle comprised of a core comprising gold and a first layer comprising zinc telluride provided on the core, preferably where the zinc telluride is capped (passivated) with cysteine, more preferably L-cysteine ethyl ester hydrochloride. In one embodiment the core consists essentially of gold and in one embodiment of the invention the cores consists of gold.

In one embodiment of the present invention the first layer consists essentially of zinc telluride and in one embodiment the first layer consist of zinc telluride.

The nanoparticle may further comprise a second layer comprising an anti-metabolite and/or antimitotic chemotherapeutic drug, a chemical tag and/or a cellular ligand, which second layer is provided on the first layer. The anti-metabolite and/or antimitotic chemotherapeutic drug may be selected from oxaliplatin, cis-platin, irinotecan, paclitaxel, 5-flouruacil and docetaxel.

The nanoparticle may be used in therapy and/or diagnosis, particularly therapy and/or diagnosis of cancer in a human or animal. Such use may include bio-imaging, tumour detection and/or thermo-destruction.

The nanoparticle may have a diameter of between 2 and 20 nanometres.

The invention further provides a method for producing a nanoparticle as hereinbefore described, the method comprising effecting conversion of the nanoparticle core precursor material, preferably gold chloride, to gold and depositing the zinc telluride layer on the gold core.

As described above, the zinc telluride is preferably capped with cysteine.

The invention further provides a method for producing metal-semiconductor nanomaterials, the method comprising the steps of:
a. Reducing a metal salt, preferably gold chloride;
b. Providing a cysteine capped zinc telluride quantum dot solution; and
c. Adding the reduced metal salt to the solution of prepared cysteine capped zinc telluride quantum dots to produce a solution of cysteine capped metal-zinc telluride core/shell nanoparticles.

The invention also provides a nano-scale drug delivery system comprising a cysteine capped gold-zinc telluride core/shell nanoparticle.

The invention extends to the use of the nanoparticle as hereinbefore described in targeted drug delivery, bio-imaging, tumour detection and/or thermo-destruction.

In this specification "quantum dots" means nanocrystals made of semiconductor materials that are small enough to exhibit quantum mechanical properties. Specifically, its excitons are confined in all three spatial dimensions. The electronic properties of these materials are intermediate between those of bulk semiconductors and of discrete molecules.

Electronic characteristics of a quantum dot are closely related to its size and shape. For example, the band gap in a quantum dot which determines the frequency range of emitted light is inversely related to its size. In fluorescent dye applications the frequency of emitted light increases as the size of the quantum dot decreases. Consequently, the colour of emitted light shifts from red to blue when the size of the quantum dot is made smaller. This allows the excitation and emission of quantum dots to be highly tunable. Since the size of a quantum dot may be set when it is made, its conductive properties may be carefully controlled. Quantum dot assemblies consisting of many different sizes, such as gradient multi-layer nanofilms, can be made to exhibit a range of desirable emission properties.

### DETAILED DESCRIPTION OF THE INVENTION

A solution-based route to biocompatible, cysteine-capped gold-zinc telluride (Au-ZnTe) core/shell nanoparticles with potential in biomedical applications is described. The optical properties of the core/shell nanoparticles show no features of the individual parent components. The tunable emission properties of the semiconductor shell render the system useful for imaging and biological labelling applications. Transmission electron microscope (TEM) imaging studies show the particles to be potentially spherical with sizes in the order of 2-10 nm. Elemental mapping using X-ray energy dispersive spectroscopy (XEDS) in the scanning transmission electron microscope (STEM) supports a core/shell morphology with Au in the core of the particle. Powder X-ray diffraction analysis revealed crystalline Au and ZnTe. The biocompatibility and cytotoxicity of the core/shells was investigated on a human pancreas adenocarcinoma (PL45) cell line using the WST-1 cell viability assay. The results showed that the core/shells had no adverse effects on the PL45 cellular proliferation or morphology. TEM image analysis confirmed the cellular uptake and isolation of the core/shell nanoparticles within the cytoplasm via membrane interactions. The fluorescence properties of the Au-ZnTe core/shells within PL45 cell lines results confirmed the bio-imaging potential of Au-ZnTe core/shell nanoparticles. The importance and novelty of this research lies in the combination of gold and zinc telluride used to produce a water soluble, biocompatible nanomaterial which may be exploited for drug delivery applications within the domain of oncology.

When gold is used as the shell component it exhibits certain advantages in playing a direct role in the size tunability and morphology of the heterostructured nanoparticle. The gold shell reduces the toxicity of the iron oxide and also enhances the biocompatibility properties. Recent studies show improvements in targeted drug delivery using gold based core/shell nanoparticles as the delivery vehicle. These studies illustrate the versatility of gold nanoparticles as either a core or shell component. The surface plasmon resonance features displayed by gold nanomaterials results in enhanced light scattering and absorption properties. These unique optical properties enable the rapid conversion of absorbed light into heat, resulting in photo thermal energy. In addition gold nanoparticles are highly stable, non-toxic and easily synthesized in a variety of sizes and surface modifications. Gold nanoparticles may be used as contrast agents in bio-imaging as they do not exhibit photo bleaching.

The inherent properties of gold nanomaterials make it a suitable core material. ZnTe was chosen as the shell component because it is a semiconductor material with a direct gap of 2.26 eV at 300 K and is an attractive material in applications which harness its optical properties. More importantly, the high stability of a gold core coupled with the luminescence properties of zinc telluride and the efficient surface modification of cysteine has the potential to enable this system to be applied in targeted drug delivery and bio-imaging applications within the domain of oncology therapeutics and diagnostics.

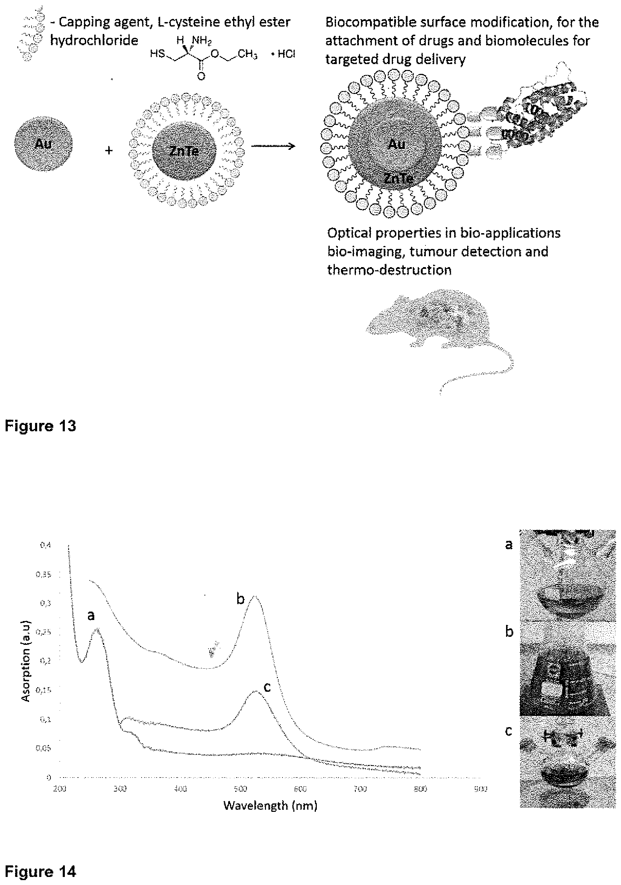

This invention teaches the synthesis of cysteine-capped Au-ZnTe core/shell nanoparticles using a simple solution-based route. Figure. 13 shows the design of core/shell nanoparticles and illustrates its potential applications. The amino functional groups on cysteine have the potential to support conjugation of cellular ligands and a combination of chemotherapeutic drugs to produce a bio-modified version of Au-ZnTe core/shell nanoparticles.

### SPECIFIC DESCRIPTION

The invention will now be described with reference to the following non-limiting figures in which:
Figure 1 shows absorption spectra of (A) cysteine capped ZnTe nanoparticles, (B) citrate capped Au nanoparticles and (C) cysteine capped Au-ZnTe core/shell nanoparticles.

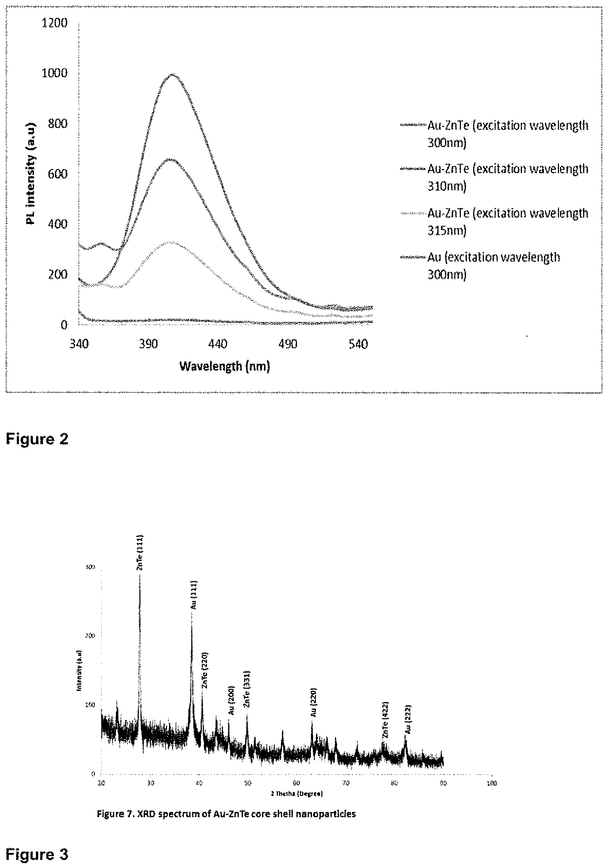

Figure 2 shows a photoluminescence spectra of Au and Au-ZnTe core-shell nanoparticles.
Figure 3 shows an X-ray diffraction pattern of Au-ZnTe core/shell nanoparticles.

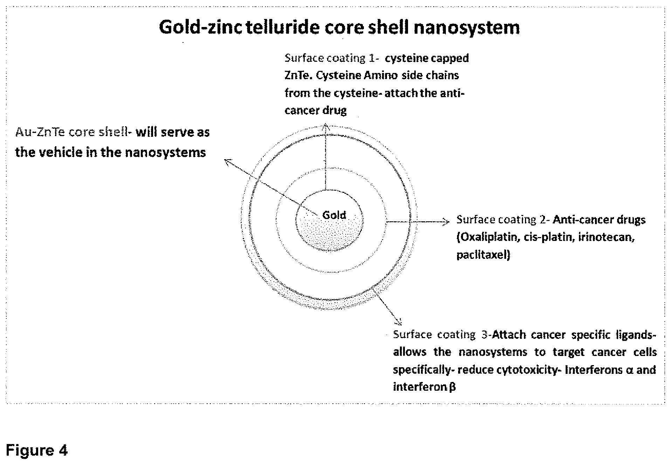

Figure 4 shows an Au-ZnTe core/shell structure with three surface coatings
Figure 5 shows a cross-section through a breast cancer cell MFC7 wherein C-cytoplasm, CM-cytoplasmic membrane, M-mitochondrion, N-nucleus, GB-golgi body and ER-endoplasmic reticulum.
Figure 6 shows a cross-section through a breast cancer cell MFC7 wherein C-cytoplasm, M-mitochondrion, N-nucleus and CM-cytoplasmic reticulum.
Figure 7 shows a cross-section through a breast cancer cell MFC7 showing the uptake of Au-ZnTe core/shell nanoparticles into the cytoplasm wherein C-cytoplasm and CM-cytoplasmic membrane.
Figure 8 shows a cross-section through a breast cancer cell MFC7 showing the uptake of Au-ZnTe core/shell nanoparticles into the cytoplasm through phagocytosis wherein C-cytoplasm and CM-cytoplasmic membrane.
Figure 9 shows a cross-section through pancreas cells PL45, showing the interaction of Au-ZnTe core/shell nanoparticles with the cellular surface wherein C-cytoplasm, CM-cytoplasmic membrane and M-mitochondria.
Figure 10 shows a cross-section through pancreas cells PL45, showing the interaction of Au-ZnTe core/shell nanoparticles with the cellular surface and nanoparticles isolated within a vacuole in the cytoplasm wherein C-cytoplasm, CM-cytoplasmic membrane and M-mitochondria.
Figure 11 shows a cross section through a MCF7 cancer cell showing nanoparticles entering the cell. The segment is enlarged showing nanoparticles entering the cell wherein C-cytoplasm, CM-cytoplasmic membrane and M-mitochondria.
Figure 12 shows a cross-section through a PL45 cell showing nanoparticles entering the cell. The segment is enlarged showing morphological features of Au-ZnTe core/shell nanoparticles wherein C-cytoplasm and CM-cytoplasmic membrane.
Figure 13 is a synthesis scheme for Au-ZnTe core/shell nanoparticles wherein the shell component is surface modified with L-cysteine ethyl ester hydrochloride for water solubility and biocompatibility features. Amino groups from the capping agent may be used to attach cell specific ligands and chemotherapeutic drugs for application in targeted drug delivery and bio-imaging.
Figure 14 shows the optical characterization of Au-ZnTe core/shell nanoparticles using UV-vis spectroscopy wherein a) cysteine capped ZnTe nanoparticles exhibit an absorbance peak at 269 nm, a brownish material is observed; b) gold nanoparticles show an absorbance peak at 519 nm, a red colloidal solution is observed; and c) Au-ZnTe core/shell nanoparticles exhibit an absorbance peak at 538 nm, a dark purple material is observed.
Figure 15 shows the optical characterization of Au-ZnTe core/shell nanoparticles using photoluminescence spectroscopy. Photoluminescence spectra of Au-ZnTe core/shell nanoparticles at various excitations are represented in a, b and c, the peak observed displays constant emission maxima at 402 nm, d. Au nanoparticles displays no emission peak for excitation at 300 nm.
Figure 16 shows the morphological characterization of Au-ZnTe core/shell nanoparticles using TEM and HRTEM wherein a) Au-ZnTe core/shell nanoparticles display spherical morphology with diameter sizes ranging between 2-10 nm and b) displays the HRTEM micrograph of Au-ZnTe core/shell nanoparticles.

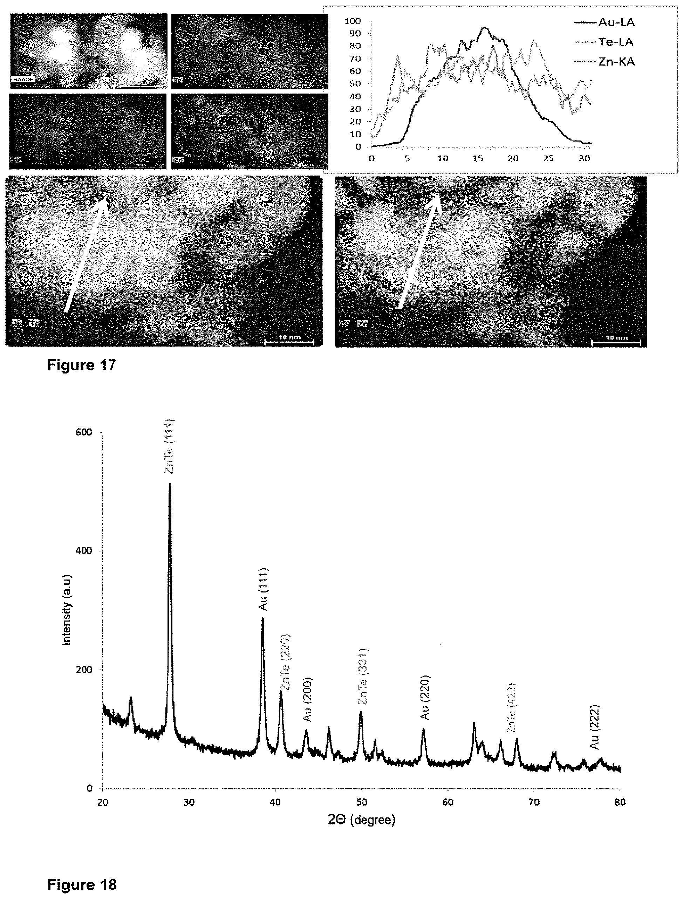

Figure 17 shows component characterisation of Au-ZnTe core/shell nanoparticles using EDX elemental mapping. A typical area of aggregated particles was chosen for analysis along with spectrum images of Au, Zn, and Te provide evidence for a core/shell structure and ratios of the heterostructured core/shell nanomaterials. Line scans taken at positions indicated are shown. Gold was found to be localised in the core with the gold distribution as shown whilst Zn and Te were observed to be principally associated with the particle surface. Line scan data extracted from the EDX spectrum images suggests a shell thickness of approximately 1 - 4 nm.
Figure 18 shows crystalline characterization of Au-ZnTe core/shell nanoparticles using XRD. The XRD spectrum displays characteristic peaks for each component material in the core/shell.
Figure 19 shows characterisation of Au-ZnTe core/shell nanoparticles using atomic force microscopy wherein a) displays the relative growth of PL45 cells after treatment with Au-ZnTe core/shell nanoparticles; b) displays the untreated control PL45 cells; c) displays PL45 cells growing at 60 % confluency under the influence of 25 µg/ml and d) displays PL45 growing at 80 % confluency under the influence of 50 µg/ml.
Figure 20 shows the cellular uptake and isolation of Au-ZnTe core/shell nanoparticles wherein a) and b) shows a cross-section through resin embedded PL45 cells, showing cellular membrane interaction with Au-ZnTe core/ shell nanoparticles; c), d), e) and f) display two adherent PL45 cells showing both membrane interactions with core/shell nanoparticles and isolation of Au-ZnTe core/shell nanoparticles within a vacuole in the cells cytoplasm. C-Cytoplasm, CM-cytoplasmic membrane and M-mitochondria.
Figure 21 shows fluorescence imaging of PL45 cancer cell lines treated with Au-ZnTe core/shell nanoparticles wherein a) displays a cross-section through pancreas cancer cell PL45 under bright field microscopy; b) shows a cross-section through pancreas cancer cell PL45 under fluorescence microscopy Dapi filter set; c) shows the fluorescence properties of Au-ZnTe core/ shell nanoparticles on different areas of the sample slides; and d) displays multi-fluorescence overlays of PL45 cells treated with Au-ZnTe core/shell nanoparticles under fluorescence Zeiss filter sets Dapi and Alexa 388.

Embodiments of the invention will now be described by means of the following examples.

### EXAMPLE 1

### Synthesis of cysteine capped gold-zinc telluride core/shell nanoparticles

An improved route to the synthesis of cysteine capped gold-zinc telluride core/shell nanoparticles is proposed. This route involves the addition of reduced gold salts to a solution of prepared cysteine capped zinc telluride quantum dots, so as to produce a dark greenish black solution of cysteine capped gold-zinc telluride core/shell nanoparticles.
In flask 1: Gold salts are reduced in the presence of citrate ions; and
In flask 2: Tellurium is reduced by sodium borohydride at room temperature under inert conditions.

The reaction is as follows:

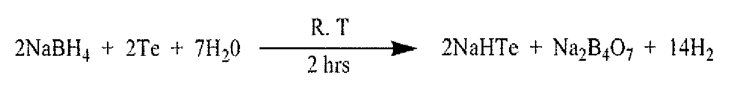

After two hours zinc chloride (zinc salt) and L-cysteine ethyl ester hydrochloride (capping agent) solutions in a 1:10 ratio are added to the pink tellurium ion solution.

The reaction is as follows;

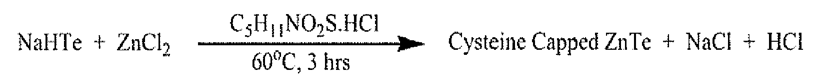

The reaction continued for 30 minutes at room temperature. The pH is adjusted to 7 and the temperature is increased to 60°C. After 3 hours the reaction is removed and added to the reduced gold solution in flask 1. After 2 hours, a greenish black material is precipitated using acetone. This product is centrifuged to give a pellet that is dried under vacuum and weighed out, to give a material that is readily dispersed in water i.e. Gold-zinc telluride core/shell nanoparticles.

These core/shell nanoparticles are further characterized using transmission electron microscope, high resolution transmission electron microscope, x-ray diffraction, ultraviolet spectroscopy and photoluminescence. This route provides highly stable water soluble cysteine capped gold-zinc telluride core/shell nanoparticles that are between 4-20 nm in diameter size.

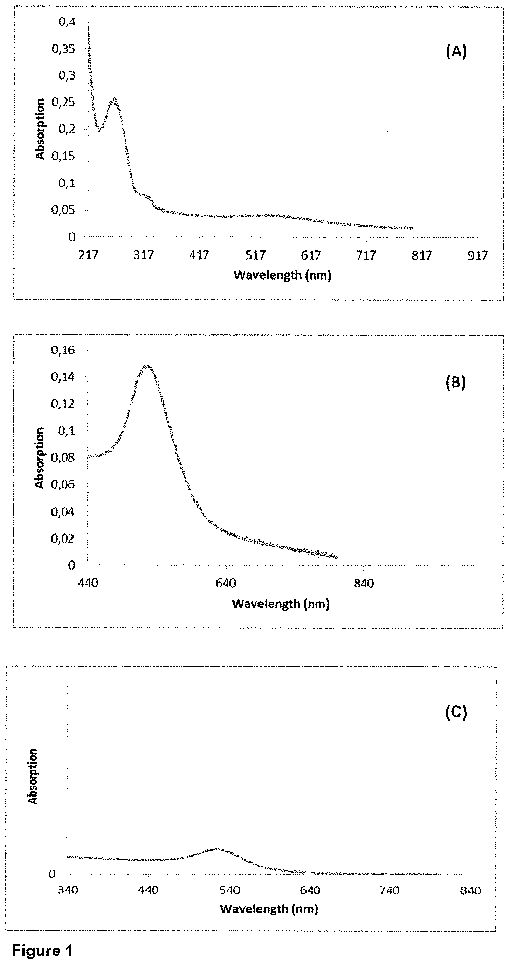

The nanoparticles thus produced showed enhanced optical properties, as illustrated in Figure 1, characterised by UV/Vis at 538 nm and PL at a range of 400 to 420 nm. The X-ray diffraction revealed characteristic peaks for both Au and ZnTe. The three peaks at 2theta = 27°, 40° and 49° corresponded to the diffraction from (111), (220) and (311) planes of cubic zinc blende ZnTe. The core showed diffraction peaks assigned to the (111), (220), (200) and (222) planes of cubic gold

The advantage of Au-ZnTe core/shells is in the design. The combination of gold core and zinc telluride shell enhances and/or shifts the optical properties. A shift in the optical property enables these core/shells to function in thermo-destruction of tumours and/or cancer tissues. The zinc telluride shell is able to function as a florescent tag for imaging of tissue if excited at the correct wavelength. These core/shell nanoparticles are surface modified or capped with cysteine. The cysteine stabilizes the core/shell complex, making Au-ZnTe core/shells water soluble and biocompatible. The amino side chain groups on the cysteine molecules provide a system for tagging drugs that can be delivered to target sites.

These core/shells were developed suitable for use as a nano drug delivery system that is cancer specific. The design allows for the attachment of existing chemotherapeutic drugs to the surface of the core/shell in addition to tumour specific receptors with the aim of creating a nanosystem for targeted drug delivery that exhibits higher efficacy.

### EXAMPLE 2

### Interaction of cysteine capped Au-ZnTe core/shell nanoparticles with cancer cell lines.

After the core/shell nanoparticles were successfully synthesized and characterized according to Example 1, they were exposed to cancer cell lines from different target organs to establish toxicity.

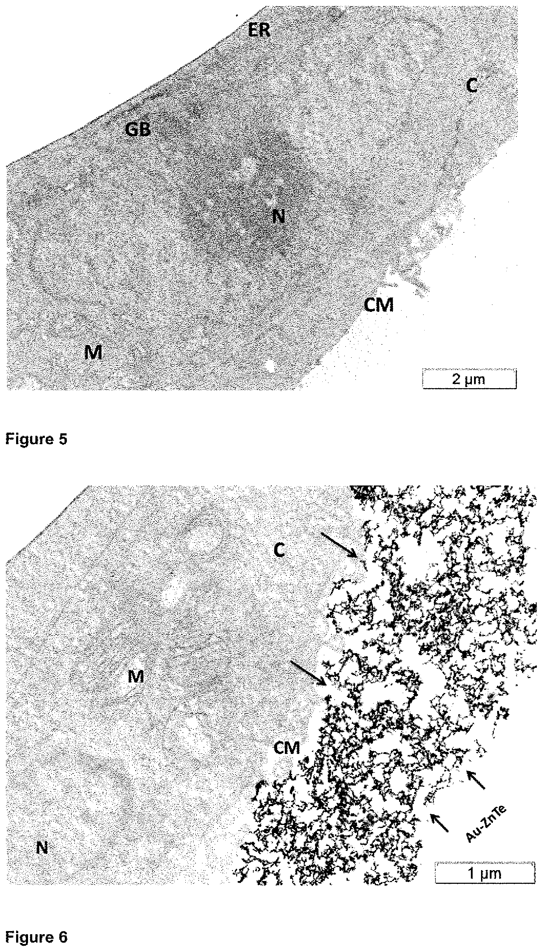

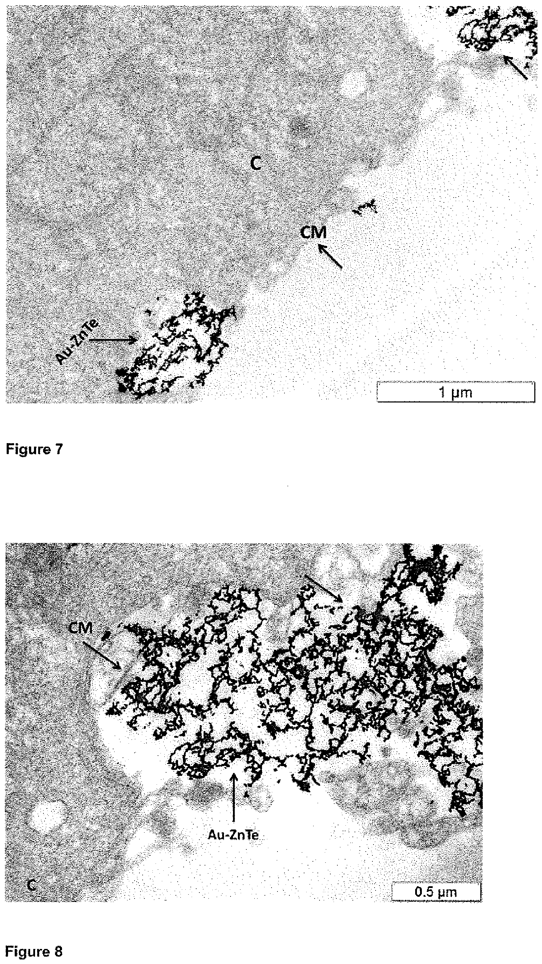

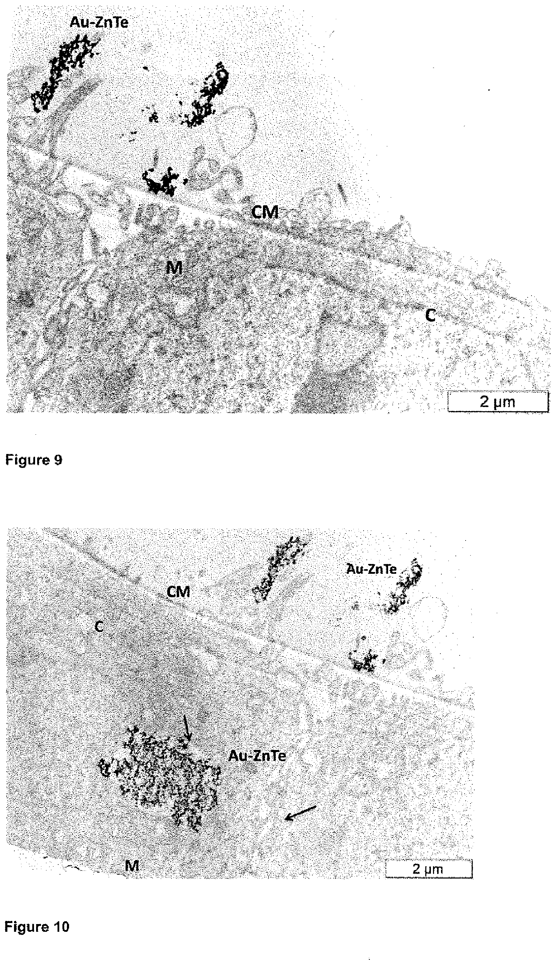

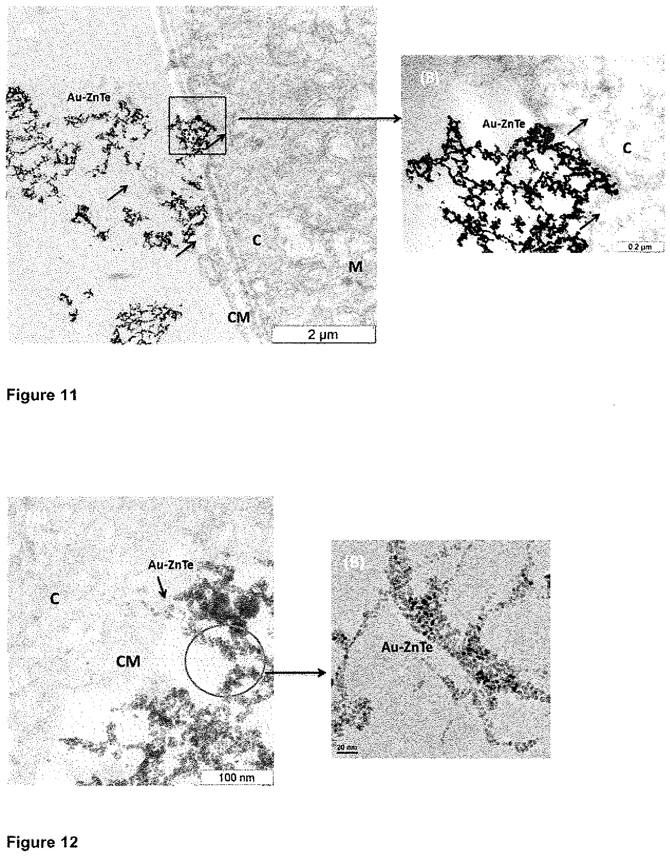

Pancreas, prostate, colon and breast cancer cell lines were exposed to different concentrations of Au-ZnTe core/shell nanoparticles and the cell viability was established. The results showed that the core/shells had no toxic effect on the growth of these cell lines. To confirm this result, cell lines from two target sites (pancreas and breast cancer) that are biochemically and morphologically different were resin embedded and micro-sectioned. The results showed that Au-ZnTe core/shell nanoparticles interact with the cytoplasmic membrane and then enter the cell through phagocytosis were they are isolated in vacuoles within the cytoplasm.
The results are illustrated in Figures 5 to 12.

### EXAMPLE 3

### Synthesis of Au-ZnTe core/shell nanoparticles

### Materials

Zinc chloride, L-cysteine ethyl ester hydrochloride, gold salt, tellurium powder, sodium borohydride, sodium citrate and deionised water (HPLC grade) and acetone were obtained from Sigma Aldrich. All the chemicals were of analytical grade and used as purchased.

### Synthesis of cysteine capped ZnTe nanoparticles

Tellurium powder (0.32 mmol) was mixed with 20 mL of deionized water in a three necked round bottom at room temperature. Sodium borohydride (0.81 mmol) was added to the reaction mixture under inert conditions. After 2 hours 20 mL of 3.2 × 10⁻⁴ M ZnCl₂ (zinc salt) and L-cysteine ethyl ester hydrochloride (capping agent) was added to the reaction mixture in molar ratios of 1:10. The reaction mixture was then heated at 60 °C for 3 hours under nitrogen gas. The cysteine capped ZnTe nanoparticles were separated from the mixture by filtration techniques and the sample was then concentrated by rotary evaporation, followed by precipitation of water soluble ZnTe nanoparticles using acetone. This product was centrifuged to give the pellet that was then dried under vacuum and weighed out to give a material that is readily dispersed in water.

### Synthesis of gold nanoparticles

The gold nanoparticles were prepared according to the procedure described by Turkevich et al. A magnetic stirrer bar was inserted into 500 ml of boiling distilled water. An aliquot of 2 ml of 1% gold chloride and 10 ml of (1.47 g of 0.5 m) sodium citrate was added to the boiling water and the reaction proceeded for 10 minutes until a dark red solution is observed.

### Synthesis of Au-ZnTe core/shell nanoparticles

In a typical room temperature reaction, tellurium powder (0.041 g, 0.32 mmol) was mixed with sterile distilled water (15.0 mL) in a three-necked round bottom flask. A 15.0 mL aqueous solution of sodium borohydride (0.031 g, 0.79 mmol) was carefully added to the tellurium solution under inert conditions (Equation 1).

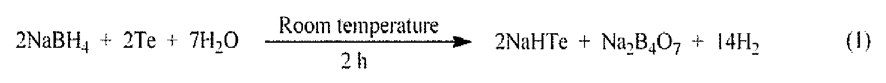

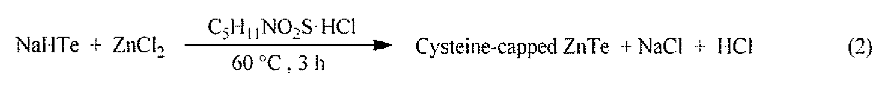

After 2 h of reduction, 20.0 mL aqueous solution of ZnCl₂ (0.0436 g, 0.32 mmol) and 40.0 mL aqueous solution of L-cysteine ethyl ester hydrochloride (0.1188 g, 0.32 mmol) was simultaneously added to the dark purple tellurium ion solution (Equation 2). The solution was stirred for 30 minutes followed by pH adjustment. The reaction was allowed to proceed at pH 7, for 3 hours at 60°C. An aliquot of 40 mL of gold nanoparticle solution was added to the cysteine capped ZnTe solution and the reaction was allowed to proceed for 4 hours. The reaction mixture was filtered and centrifuged to give a dark greenish black material that was readily dispersed in sterile distilled water.

### Characterisation of nanoparticles

A Perkin-Elmer Lamda 20 UV-vis spectrophotometer was used to carry out optical measurements in the 200-1100 nm wavelength range at room temperature. Samples were placed in quartz cuvettes (1 cm path length) and the absorbance was recorded. Photoluminescence (PL) spectra were recorded on a Perkin-Elmer LS 55 luminescence spectrometer with xenon lamp over range of 200-800 nm. The samples were placed in quartz cuvettes (1 cm path length) and the excitation peaks were analysed and recorded. Samples for S/TEM imaging and spectroscopic analysis were prepared by drop casting an aliquot solution of Au-ZnTe nanoparticle material onto a TEM support grid (consisting of a holey carbon amorphous film supported on a copper mesh) and then allowing the solvent to evaporate at room temperature. Preliminary TEM imaging of the Au, ZnTe and the Au-ZnTe nanoparticles was performed using a Philips CM120 BIOTWIN TEM operated at 120kV. A JEOL JEM 3010 URP high resolution transmission electron microscope (HRTEM) operated at 300 kV was used for high resolution imaging of the Au-ZnTe samples. High angle annular dark field (HAADF) imaging was performed in Titan G2 80-200 ChemiSTEMTM scanning transmission electron microscope (STEM) operated at 200 kV with a convergence angle of 18mrad and a HAADF inner angle of 54 mrad. X-ray energy dispersive spectroscopy (XEDS) was performed using the Titan's Super-X X-ray detector system which has a solid collection angle of 0.7 srad. XRD measurements of the Au-ZnTe samples were performed using a Bruker aXS D8 advanced diffractometer with Cu-Kα radiation (λ= 1.5406 Å) operated at 40 kV and 40 mA. TEM imaging of the cellular uptake of nanoparticles was performed using a Philips CM120 BIOTWIN TEM operated at an accelerating voltage of 120 kV with the sample cooled to 80K. The Zeta potential of the core/shell nanoparticles was determined by a Zetasizer, Nanoseries, Malvern Instrument. Samples were filtered several times through a 0.22 mm Millipore membrane filter prior to recording surface charge measurements. Fluorescence images were recorded using a Carl Zeiss Axio Imager 2 fluorescence and phase-contrast microscope. The cross-section of PL45 cell sample was placed on a slide and viewed under a Dapi and Alexa filter set.

Au-ZnTe core/shell nanoparticles were synthesized as described above using a novel one pot method under an inert atmosphere. A dark purple colour was observed after 2 hours, indicating the reduction of tellurium powder. The solution turned dark brownish to black in colour after the addition of zinc chloride and L-cysteine ethyl ester hydrochloride. The material from this reaction was purified for characterisation analysis. The absorption properties of the parent and the core/shell material were measured. The cysteine capped ZnTe nanoparticles showed an absorption peak at 269 nm (Fig. 14a) which is attributed to the Zn-ligand complex as observed previously for cysteine capped CdTe. The gold nanoparticles exhibited their characteristic surface plasmon resonance (SPR) absorbance at approximately 515 nm (Fig. 14b). The absorption spectra of the Au-ZnTe nanoparticles showed an absorption peak at 538 nm (Fig. 14c), a red shift in relation to the absorption of the gold. The position and shape of the absorption of the Au-ZnTe core/shell system peak shows no resemblance to that of the parent materials.

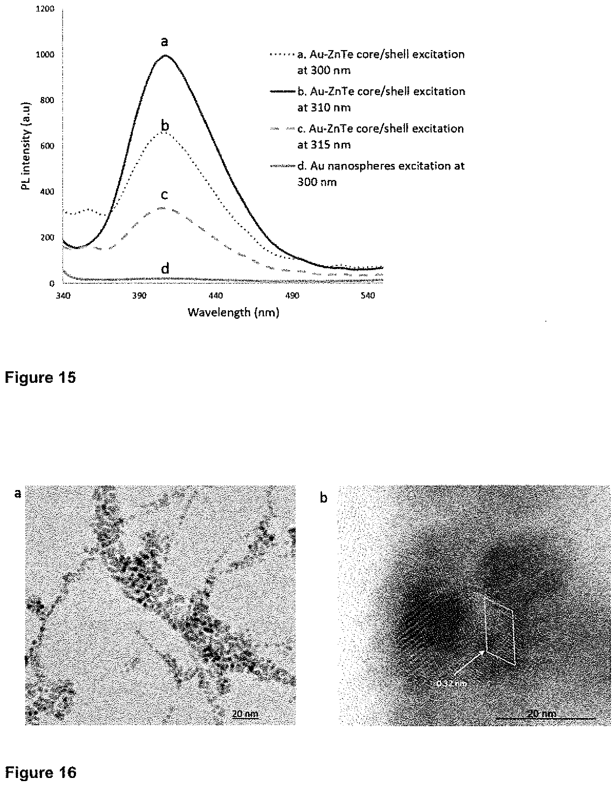

The photoluminescence properties of Au-ZnTe core/shell nanoparticles were studied (Fig 15). The Au-ZnTe core/shell nanoparticles showed sharp, Gaussian shaped emissions. Excitation at different wavelengths (300, 310 and 320 nm) produced a constant peak at 402 nm in the PL spectra (Fig.15 a, b and c). Previous reports on ZnTe nanoparticles show emission in the 400-600 nm range.

TEM images of Au-ZnTe showed spherical particles with diameters in the 2-10 nm range. The self-assembled arrangement of the Au-ZnTe nanoparticles on the carbon grid is similar to that reported for Au-CdSe. Agglomerated clusters of regular shaped spherical particles with dark gold cores and more transparent ZnTe shells were observed in high resolution TEM analysis. The HRTEM images showed particles with lattice fringes. The lattice spacing of 0.32 nm observed in (Fig. 16 b), is assigned to the {111} plane of cubic ZnTe.

HAADF-STEM and EDX spectrum were used to confirm the structure and ratio of the heterostructured core/shell nanomaterials. The data obtained from this study (displayed in Fig.17) confirm the core/shell structure of the Au-ZnTe core/shell nanoparticles. Gold was observed as a core material whilst ZnTe was observed as the shell material with a thickness of ±2 nm. A comparative line scan of the selected area in the sample shows the ratios of Au, Zn and Te in the core/shell nanoparticles.

The powder X-ray diffraction pattern of the Au-ZnTe nanoparticles is shown in (Fig. 18 a). The pattern reveals characteristic peaks for both Au and ZnTe. The three peaks at 2θ = 27°, 40° and 49° correspond to the expected lattice spacing of the {111}, {220} and {311} planes of the cubic zinc blended ZnTe structure. The peaks at 2θ = 38°, 46°, 65° and 78° are assigned to the {111}, {220}, {200} and {222} lattice planes of cubic gold. It was not possible to account for the unassigned peaks that were observed in the XRD pattern. The formation of ZnS as a secondary nanomaterial was considered as reported previously for cysteine capped CdTe whereby the sulfur from the cysteine is hydrolysed and reacts with cadmium to form an additional CdS shell. No evidence was found from the XRD diffraction pattern for any ZnS formation through the reaction of sulfur from cysteine. It may be possible that some of the unassigned peaks could be attributed to unreacted tellurium from the reaction.

### EXAMPLE 4

### Cellular uptake of Au-ZnTe core/shell nanoparticles

One of the key attractions of the Au-ZnTe nanoparticle system lies in the applications that arise. The biocompatibility of this core/shell system was investigated with human cancer cell lines from various organs. This example is restricted to a human pancreas adenocarcinoma PL45 cell line that was co-cultured with Au-ZnTe core/shell nanoparticles. The cellular toxicity was studied using the WST-1 assay which is based on the cleavage of water soluble tetrazolium salt to a formazan dye by succinate-tetrazolium reductase, this reaction occurs in the mitochondrial respiratory chain and is active only in viable cells.

### Tissue culture media and cell line

Dulbecco's modified eagle medium (DMEM), Dulbecco's Phosphate buffered saline (PBS), Trypsin, 100x pen-strep Fungizone (PSF) and fetal calf serum (FCS). All the cell tissue culture reagents were supplied by Invitrogen. Human pancreas adenocarcinoma cell line PL45, was purchase from American Type Culture Collection (ATCC) and used in the experiments. This adhesion cell line was grown in DMEM medium containing 10% FBS and 1-2% PSF at a cell density of 5000. Cells that grew as monolayers were passaged, trypsinized and harvested for experimentation before reaching 100 % confluency.

### WST-1 cell viability assay

Cell viability was evaluated by WST-1 (4-[3-(4-iodophenyl)-2-(4-nitrophenyl)-2H-5-tetrazolio]-1,3-benzene disulfonate) assay (Roche diagnostics, Mannheim, Germany) according to the manufacturer's instructions. Nanoparticle treated and control cells were seeded in a 96 well plate at a cell density of 105 per well in 100 µl of appropriate culture media. Cells were cultured in a CO₂ at 37°C for 48 hours. Reconstituted WST-1 reagent was added to each treatment well and incubated for 2 hours at 37°C. The plate was shaken for 30 seconds and the absorbance was measured at 450 nm using a Multiskan FC microplate reader. All experiments were repeated in triplicates and the results were represented as mean SEM (standard error of the mean) using the following formula: (Test sample)/(Solvent control)*100=% cell viability

### Cellular uptake of Au-ZnTe core/shell nanoparticles

PL45 cell lines were treated with Au-ZnTe core/shell nanoparticles for 48 hours at the following concentrations, 3.125, 6.25, 12.5, 25 and 50 ug/ml. The treated cells were then fixed with 2.5 % glutaraldehyde for 24 hours, followed by a phosphate buffer wash. A postfix of 0.5 % osmium tetroxide was added to the cells and incubated for 1 hour at room temperature followed by a phosphate buffer wash. The cells were then dehydrated with 30, 50, 75 and 100 % acetone. Resin and acetone were added to the sample in equal volumes followed by 4 hour incubation at room temperature. Cells were placed in moulds and whole resin, followed by an overnight incubation at 70 °C. Resin sample blocks were sectioned into ultrathin cross sections using a microtome, stained and viewed with a transmission electron microscope.

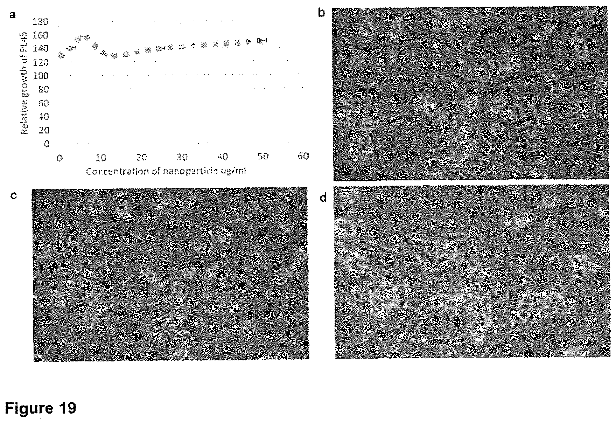

PL45 cells showed no dose-dependent inhibition on cell proliferation after 72 hours of exposure to Au-ZnTe core/shell nanoparticles at the following concentrations 3.125, 6.25, 12.5. 25 and 50 ug/ml (Fig. 19a). A comparison of cellular morphology after exposure to Au-ZnTe core/shell nanoparticles is displayed in (Fig 19 b, c and d). No significant morphology variations were observed in the pancreas cancer cell line.

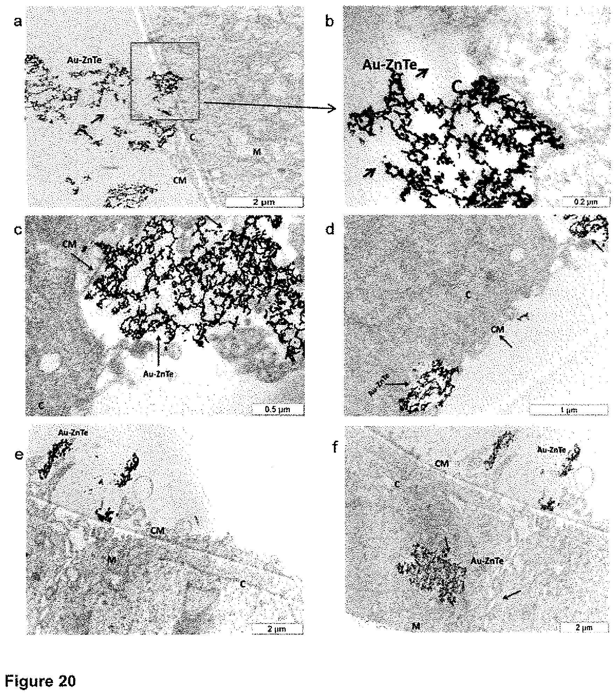

The interaction of Au-ZnTe core/shell nanoparticles and PL45 was investigated using embedding, ultra-thin microtome sectioning and TEM imaging. Cellular uptake and isolation of the core/shell nanoparticles through membrane interactions was confirmed. The cysteine surface layer may have been responsible for the membrane interactions with the core/shell nanoparticles (Fig. 20 a-f). The process of nanoparticle uptake and vacuole isolation is clearly visualized. The mechanism by which the nanomaterial enters the cellular membranes is still under enquiry.

The surface charge of nanomaterials is an important factor that may be altered by the attachment of bioconjugates. Monitoring the surface charge allows control in electrostatic and hydrophobic interactions within biological systems resulting in intracellular targeting and drug carrier applications. The measurement of the Zeta potential was used to determine the surface charge of the parent precursors, Au-ZnTe core/shell and cysteine. In addition it was used to determine how efficiently the capping agent has passivated the outermost surface of the Au-ZnTe core/shell nanoparticles. The Au and ZnTe parent precursors displayed negative charges of - 65.1 mV and -11.7 mV respectively. Interestingly the Au-ZnTe core/shell nanoparticles displayed a positive close to neutral charge of 0.0519 mV. Cysteine displayed a positive charge of 3.95 mV. The results confirmed that the capping agent influenced the surface charge of Au-ZnTe and that the core/shell nanoparticles are efficiently passivated by an outer capping layer of cysteine. The surface charge of the core/shell nanoparticles will accommodate the functionalization of this nanomaterial for further applications in cell targeting and therapeutics.

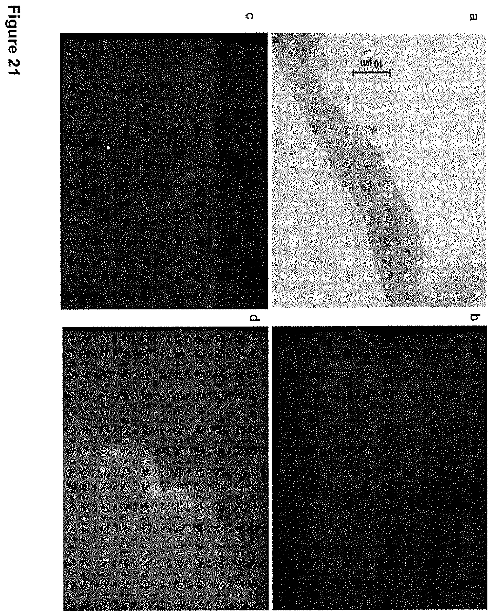

The fluorescence properties of Au-ZnTe core/shell nanoparticles within the PL45 cell lines were studied using fluorescence and phase-contrast microscopes. Fig 21 a. shows the cross section of resin embedded PL45 cells treated with Au-ZnTe core/shell nanoparticles under a bright field view. Fig 21.b, shows the same slide under a Dapi fluorescent filter set. The results illustrate imaging by Au-ZnTe core/shell nanoparticles. The blue light emissions correspond to the absorption wavelength between 365 - 415 nm and is due to emission from the ZnTe shell layer. Figs 21 c and d display various sections on the sample slide that are fluorescing due to the presence of the core/shell nanoparticles under Dapi and Alexa 388 filter sets. The focus of this study was optimising the synthetic procedures and to understand the features of Au-ZnTe core/shell nanoparticles. Modification of the core/shell surface with cysteine allowed the particles to be water soluble as well as biocompatible. The amino functional groups of cysteine can be utilized for the attachment of biological receptors, tags or ligands. The chain-like arrangement observed for Au-ZnTe core/shell nanoparticles is characteristic of cysteine capped gold nanoparticles. In their study, Horovitz and co-workers investigated the assembly of gold nanoparticles which were induced by cysteine, an amino acid possessing an additional thiol functional group besides the alpha-amine.50 The cysteine assembly effect was explained by considering the zwitterion-type electrostatic interactions between the charged amine and acid groups of cysteine molecules, bound to the gold nanoparticles by their -SH groups. They also did not rule out the interaction between cysteine and citrate bound to gold nanoparticles. In this work the cysteine is bound to the ZnTe shell with electrostatic interactions as well as with the citrate covered gold forming a network or assembly of particles.

Previous reports on Au particles covered with metal shells showed that this surface plasmon absorption peak can be tuned by changing the thickness of the shell. Lu et al, demonstrated that the plasmon absorption of gold gradually shifts until a peak characteristic of the shell material emerges. The optical properties displayed by Au-ZnTe core/shell nanoparticles provide evidence that the ZnTe shell is fluorescent. The emission from the Au-ZnTe nanoparticles could be useful in biomedical applications such as tumour imaging. The emission properties can be harnessed by tracking the movement of the Au-ZnTe drug coated particles within cells or monitoring tumour size after drug treatments. This may well be more efficient than the use of organic dyes as labels. Reports of cytotoxicity and biocompatibility indicate that Au-ZnTe core/shell nanoparticles are biocompatible. These results provide a platform for further studies to investigate the mechanistic interactions between the cellular membranes and core/shell nanoparticles and also to venture into the research of surface attachment of complimentary drugs and cellular ligands. The invention therefore extends to the use of Au-ZnTe core/shell nanoparticles as a template system for the attachment of various anti-metabolite and anti-mitotic chemotherapeutic drugs such as 5-fluoruracil, docetaxel and paclitaxel. The use of a combination of chemotherapy drugs on a core/shell system will facilitate enhanced pharmacokinetics and therapeutic efficacy. In addition cellular receptors that are presented on various types of cancer cells may be attached to the biocompatible core/shell surface to facilitate therapy that is targeted specifically to cancer cells.

The present invention has optimized a synthesis route to produce water soluble Au-ZnTe core/shell nanoparticles with distinct features. Cellular uptake studies confirmed the biocompatibility of the core/shells within cellular systems.

### References

1. Danhier. F., Feron. O., Préat. V. To exploit the tumor microenvironment: Passive and active tumor targeting of nanocarriers for anti-cancer drug delivery. J. Controlled Release. 148, 135-146(2010).
2. Vigderman. L and Zubarev. E.R. Therapeutic platforms based on gold nanoparticles and their covalent conjugates with drug molecules. Adv. Drug. Deliv. Rev. 65,663-676 (2013).
3. Chaudhuri, R.G. and Paria, S. Core/Shell Nanoparticles: Classes, Properties, Synthesis Mechanisms, Characterization, and Applications Chem. Rev., 112, 2373-2433 (2012)
4. Huang, X., Jain P.K., El-Sayed, I.H., El-Sayed, M.A. Gold nanoparticles: interesting optical properties and recent applications in cancer diagnostics and therapy. Nanomedicine. 2(5), 681-693 (2007)
5. Hsu, C. Huang, C. Hao, Y. Liu, F. Au/Pd core/shell nanoparticles with varied hollow Au cores for enhanced formic acid oxidation. Nanoscale Res Lett. 8:113 (2013)
6. Mitsudome, T. and Kaneda, K. Advanced Core-Shell Nanoparticle Catalysts for Efficient Organic Transformations ChemCatChem. 5, 1681-1691 (2013).
7. Habas, S.E Lee, H. Radmilovic V., Somorjai, G.A Yang, P. Shaping binary metal nanocrystals through epitaxial seeded growth. Nat.Mater. 6, 692-697 (2007).
8. Slater, M.A., Schroeder, S.L.M., Burke. M.G., O'Brien. P., Carmargo. P.H.C., Haigh. S.J. Correlating catalytic activity of Ag-Au nanoparticles with 3D compositional variations. Nano Lett. 14 (4), 1921-1926 (2014).
9. Lee, J.S., Shevchenko, E.V., Talapin. D.V. Au-PbS core/shell nanocrystals: plasmonic absorption enhancement and electrical doping via intra-particle charge transfer. J. Am. Chem. Soc. 130, 9673 (2008).
10. Li, J., and Zeng, H.C. Size tuning, functionalization, and reactivation of Au in TiO2 nanoreactors. Angew. Chem. Int. Ed. 44, 4342-4345 (2005).
11. Liu, M.Z., Guyot-Sionnest, P. Preparation and optical properties of silver chalcogenide coated gold nanorods. J. Mater. Chem. 16, 3942-3945 (2006).
12. Shevchenko, E.V., Bodnarchuk, M.I., Kovalenko, M.V, Talapin, D.V, Smith, R.K., Aloni, S., Heiss, W. A. P. Alivisatos. Tuning the Magnetic Properties of Metal Oxide Nanocrystal Heterostructures by Cation Exchange. Adv.Mater. 20, 4323-4329 (2008).
13. Qian, L., Sha,Y., Yang, X. Simple and convenient preparation of Au-Pt core/shell nanoparticles on surface via a seed growth method. Thin Solid Films. 515 (4), 1349-1353 (2006).
14.Sun, Z., Yang, Z., Zhou, J., Yeung, M.H., Ni W., Wu, H., Wang, J. A General Approach to the Synthesis of Gold-Metal Sulfide Core-Shell and Heterostructures. Angew. Chem. Int. Ed. 48, 2881 -2885 (2009).
15. Salant, A., Amitay-Sadovsky, E., Banin, U.Directed Self-Assembly of Gold-Tipped CdSe Nanorods. J. Am. Chem. Soc. 128, 10006-10007 (2006).
16. Mokari, T., Sztrum C.G., Salant, A. Rabani, E. Banin, U.Formation of Asymmetric One-Sided Metal Tipped Semiconductor Nanocrystal Dots and Rods. Nat. Mater. 4, 855-863 (2005).
17. Mokari, T., Rothenberg E., Popov, I., Costi R., Banin, U.Selective Growth of Metal Tips onto Semiconductor Quantum Rods and Tetrapods. Science, 304, 1787-1790 (2004).
18. Goon I.Y., Lai, L.M.H., Lim, M., P. Munroe, Gooding,J.J., Amal, R. Fabrication and Dispersion of Gold-Shell-Protected Magnetite Nanoparticles: Systematic ControlUsing Polyethyleneimine. Chem. Mat. 21, 673-681 (2009).
19. Zhang, J., Post, M., Veres, T., Jakubek, Z.J., Guan, J., Wang, D., Normandin, F., Deslandes, Y., Simard, B. Laser-assisted synthesis of superparamagnetic Fe@Au core/shell nanoparticles. J. Phys. Chem. B. 110, 7122-7128 (2006).
20. Bao, Y.P., Calderon, H., Krishnan, K.M. Synthesis and Characterization of Magnetic-Optical Co-Au Core/shell Nanoparticles. J. Phys. Chem. C. 111, 1941-1944 (2007).
21. Pal, S., Morales, M., Mukherjee, P., Srikanth H. Synthesis and Magnetic Properties of Gold-Coated Iron Oxide Nanoparticles. J. Appl. Phys.2009, 105, 07B504 (2009).
22. Lim, J., Eggeman, A., Lanni, F., Tilton, R.D., Majetich, S.A. Synthesis and single-particle optical detection of low-polydispersity plasmonic-superparamagnetic nanoparticles. Adv. Mater. 20, 1721-1726 (2008).
23. Chiang, I.C. and Chen, D.H. Synthesis of Monodisperse FeAu Nanoparticles with Tunable Magnetic and Optical Properties. Adv. Funct. Mater. 17, 1311-1316 (2007).
24. Wei, Y.H., Klajn, R., Pinchuk, A.O., Grzybowski, B.A. Synthesis, Shape Control, and Optical Properties of Hybrid Au/Fe3O4 Nanoflowers. Small. 4, 1635-1639 (2008).
25. Melancon, M. Lu, W., Li. C. Gold-Based Magneto/Optical Nanostructures: Challenges for In Vivo Applications in Cancer Diagnostics and Therapy. Mater Res Bull. 34 (6), 415-421 (2009).
26. Kayal. S. and Ramanujan. V.R. Anti-Cancer Drug Loaded Iron-Gold core-shell nanoparticles (Fe@Au) for Magnetic Drug Targeting. J Nanosci Nanotechnol. 10, 1-13(2012).
27. Pullabhotla,V.S.R. and Revaprasadu, A novel route to cysteine capped Au-CdSe hybrid nanoparticles N. Mater. Lett. 63, 2097-2099 (2009)
28. Duncan, B., Kim, C., Rotello, V.M. Gold nanoparticle platforms as drug and biomacromolecule delivery systems. J Control Release. 148, 122-127 (2010).
29. Jain, P.K., El-Sayed I.H., El-Sayed M.A. Au nanoparticles target cancer. Nanotoday. 2 (1), (2007).
30. Crowder, B.L., Morehead, F.F., Wagner, P.R. Efficient Injection Electroluminescence in ZnTe by Avalanche Breakdown. Appl. Phys. Lett. 8, 148-149 (1966).
31. Sou, I.K., Wong, K.S., Yang, Z.Y. Wang, H., Wong, G.K.L. Highly efficient light emission from ZnSTe alloy. Appl.Phys. Lett. 66, 1915-1917 (1995).
32. Wu, Q., Litz, M., Zhang, X.C. Broadband detection capability of ZnTe electro-optic field detectors. Appl. Phys. Lett. 68, 2924-2926 (1996).
33. Bhunia, S., and Bose, D.N. Microwave synthesis, single crystal growth and characterization of ZnTe. J. Cryst. Growth. 186, 535-542 (1998).
34. Mntungwa, N., Pullabhotla, V.S.R., Revaprasadu, N. Facile synthesis of cysteine and triethanolamine capped CdTe nanoparticles. Colloids Surf., B. 101, 450-456 (2013).
35. Jiang, F., Li, Y., Ye, M., Fan, L., Ding, Y., Li, Y. Ligand-Tuned Shape Control, Oriented Assembly, and Electrochemical Characterization of Colloidal ZnTe Nanocrystals. Chem. Mater. 22, 4632-4641 (2010).
36. Kang. S.W., Lee. Y.W., park. Y., Choi. B., Hong. J.W., Park. K., Han. S.W. One-Pot Synthesis of Trimetallic Au@PdPt Core/Shell Nanoparticles with High Catalytic Performance. ACS Nano. 7, (9) 7945-7955 (2013).
37. L'Azou . B., Jorly. J., On. D., Sellier. E., Moisan. F., Fleury-Feith. J., Cambar. J., Brochard. P., Ohayon-Courtès. C. In vitro effects of nanoparticles on renal cells. Part. Fibre toxicol. 5: 22. (2008).
38. Gibson, J.D., Khanal, B.P., Zubarev. E.R. Paclitaxel-Functionalized Gold Nanoparticles. J. Am. Chem. Soc. 129, 11653-11661 (2007).
39. Patra, C.R., Bhattacharya, R., Mukhopadhyay, D., Mukherjee, P. Fabrication of Gold Nanoparticles for targeted therapy in pancreatic cancer. Adv. Drug. Deliv. Rev. 62(3), 346-361 (2010).
40. Pujalte I., Passagne I., Brouillaud B., Tréguer M., Durand E., Ohayon-Courtès C., L'Azou B. Cytotoxicity and oxidative stress induced by different metallic nanoparticles on human kidney cells Part. Fibre toxicol. 8:10 (2011).
41. Parveen, S., Misra, R., Sahoo, S.K. Nanoparticles: a boon to drug delivery, therapeutics, diagnostics and imaging. Nanomedicine. 8, 147-166 (2012).
42. Lianga, H., Chenb, C., Chena, S., Kulkarnia, A.R., Chiua, Y., Chena, M., Sung, H. Paclitaxel-loaded poly(g-glutamic acid)-poly(lactide) nanoparticles as a targeted drug delivery system for the treatment of liver cancer. Biomaterials. 27, 2051-2059 (2006).
43. Iversen, T., Skotlanda, T., Sandvig, K. Endocytosis and intracellular transport of nanoparticles: Present knowledge and need for future studies. Nano Today. 2011. 6, 176-185 (2011).
44. Labhasetwar. V. Nanotechnology for drug and gene therapy: the importance of understanding molecular mechanisms of delivery. Curr. Opin. Biotechnol. 16, 674-680 (2005).
45. Mocanu, A., Cernica, I., Tomoaia, G., Bobos, L., Horovitz, O., Tomoaia-Cotisel, M. Self-assembly characteristics of gold nanoparticles in the presence of cysteine. Colloids and Surfaces A: Physicochem. Eng. Aspects. 338, 93-101 (2009).
46. Zeta potential: a surface electrical characteristic to probe the interaction of nanoparticles with normal and cancer human breast epithelial cells. Biomed Microdevices. 10,321-328 (2008).
47. A. Valizadeh, H. Mikaeili, M. Samiei, S. M. Farkhani, N. Zarghami, M. kouhi, A. Akbarzadeh, S. Davaran. Quantum dots: synthesis, bioapplications, and toxicity. Nanoscale Res Lett. 7, 480 (2012).
48. L. Shao, Y. Gao, F. Yan. Semiconductor Quantum Dots for Biomedical Applications. Sensors. 11, 11736-11751 (2011).
49. Hao. X., Zhou. M., Zhang. X., Yu. J., Jie. J., Yu. C., Zhang. X. Highly luminescent and photostable core-shell dye nanoparticles for high efficiency bioimaging. Chem. Commun. 50, 737-739 (2014).
50. Lu. L., Burkey. G., Halaciuga. I., Goia. D.V. Core-shell gold/silver nanoparticles: Synthesis and optical properties. Journal of Colloid and Interface Science. 392, 90-95 (2013).
51. Kimling. J., Maier. M., Okenve. B., Kotaidis. V., Ballot. H., Pleach, A. Turkevich method for gold nanoparticle synthesis revisited. J Phys Chem B. 110(32), 15700-15707 (2006).

## Claims

1. A nanoparticle comprised of a core comprising gold and a first layer comprising zinc telluride provided on the core, wherein the zinc telluride is capped with cysteine.

2. A nanoparticle according to claim 1 wherein the zinc tellurium is capped with L-cysteine ethyl ester hydrochloride.

3. A nanoparticle according to claim 1 or 2 further comprising a second layer comprising an anti-metabolite and/or antimitotic chemotherapeutic drug, a chemical tag and/or a cellular ligand, which second layer is provided on the first layer.

4. A nanoparticle according to claim 3 wherein the anti-metabolite and/or antimitotic chemotherapeutic drug is selected from oxaliplatin, cis-platin, irinotecan, paclitaxel, 5-flouruacil and docetaxel.

5. Ananoparticle according to claim 1 for use in therapy and/or diagnosis.

6. A nanoparticle according to claim 5 for use in therapy and/or diagnosis of cancer in a human or animal.

7. A nanoparticle according to claim 6 for use in bio-imaging, tumour detection and/or thermo-destruction.

8. A nanoparticle according to any one of claims 1 to 7 having a diameter of between 2 and 20 nanometres.

9. A method for producing a nanoparticle according to any one of claims 1 to 8, the method comprising effecting conversion of the nanoparticle core precursor material to gold, depositing the zinc telluride layer on the gold core and capping the zinc telluride with cysteine.

10. The method according to claim 9 wherein the nanoparticle core precursor material is gold chloride.

11. A method for producing a cysteine capped gold-zinc telluride nanomaterial, the method comprising the steps of:
a. reducing a gold salt;
b. adding reduced telluirium, zinc salt and cysteine to form a cysteine capped zinc telluride quantum dot solution; and
c. adding the reduced gold salt to the solution of prepared cysteine capped zinc telluride quantum dots to produce a solution of cysteine capped gold-zinc telluride core/shell nanoparticles.

12. A method according to claim 13 wherein the gold salt is gold chloride.

13. A nano-scale drug delivery system comprising a cysteine capped gold-zinc telluride core/shell nanoparticle.

## Patentansprüche

1. Nanopartikel, bestehend aus einem Kern aus Gold und einer ersten Schicht, die Zinktellurid umfasst, das auf dem Kern vorgesehen ist, wobei das Zinktellurid mit Cystein verkappt ist.

2. Nanopartikel nach Anspruch 1, wobei das Zinktellur mit L-Cysteinethylester-hydrochlorid verkappt ist.

3. Nanopartikel nach Anspruch 1 oder 2, der ferner eine zweite Schicht umfasst, die ein antimetaboles und/oder antimitotisches chemotherapeutisches Medikament, eine chemische Markierung und/oder einen zellulären Liganden umfasst, wobei die zweite Schicht auf der ersten Schicht vorgesehen ist.

4. Nanopartikel nach Anspruch 3, wobei das antimetabole und/oder antimitotische chemotherapeutische Medikament ausgewählt ist aus Oxaliplatin, cis-Platin, Irinotecan, Paclitaxel, 5-Fluoruracil und Docetaxel.

5. Nanopartikel nach Anspruch 1 zur Verwendung in der Therapie und/oder Diagnose.

6. Nanopartikel nach Anspruch 5 zur Verwendung in der Therapie und/oder Diagnose von Krebs bei einem Menschen oder Tier.

7. Nanopartikel nach Anspruch 6 zur Verwendung beim Bioimaging, Tumor-Nachweis und/oder bei der Thermozerstörung.

8. Nanopartikel nach einem der Ansprüche 1 bis 7 mit einem Durchmesser zwischen 2 und 20 Nanometern.

9. Verfahren zur Herstellung eines Nanopartikels nach einem der Ansprüche 1 bis 8, wobei das Verfahren das Bewirken einer Umwandlung des Nanopartikelkern-Vorläufermaterials zu Gold, das Ablagern der Zinktellurid-Schicht auf dem Goldkern und das Verkappen des Zinktellurids mit Cystein umfasst.

10. Verfahren nach Anspruch 9, wobei das Nanopartikelkern-Vorläufermaterial Goldchlorid ist.

11. Verfahren zur Herstellung eines mit Cystein verkappten Gold-Zinktellurid-Nanomaterials, wobei das Verfahren die folgenden Schritte umfasst:
a. Reduzieren eines Goldsalzes;
b. Zugeben von reduziertem Tellur, Zinksalz und Cystein zur Bildung einer mit Cystein verkappten Zinktellurid-Quantenpunkt-Lösung; und
c. Zugeben des reduzierten Goldsalzes zu der Lösung von zubereiteten mit Cystein verkappten Zinktellurid-Quantenpunkten zur Herstellung einer Lösung von mit Cystein verkappten Gold-Zinktellurid-Kern/Schalen-Nanopartikeln.

12. Verfahren nach Anspruch 13, wobei das Goldsalz Goldchlorid ist.

13. Nanoskaliges Medikamentabgabesystem, das einen mit Cystein verkappten Gold-Zinktellurid-Kern/Schalen-Nanopartikel umfasst.

## Revendications

1. Nanoparticule se composant d'un noyau comprenant de l'or et d'une première couche comprenant du tellurure de zinc disposée sur le noyau, dans laquelle le tellurure de zinc est coiffé avec de la cystéine.

2. Nanoparticule selon la revendication 1, dans laquelle le tellurure de zinc est coiffé avec du chlorhydrate d'ester éthylique de L-cystéine.

3. Nanoparticule selon la revendication 1 ou 2, comprenant en outre une seconde couche comprenant un médicament chimiothérapeutique antimétabolite et/ou antimitotique, une étiquette chimique et/ou un ligand cellulaire, ladite seconde couche est disposée sur la première couche.

4. Nanoparticule selon la revendication 3, dans laquelle le médicament chimiothérapeutique antimétabolite et/ou antimitotique est sélectionné parmi l'oxaliplatine, le cisplatine, l'irinotécan, le paclitaxel, le 5-fluoruacil et le docétaxel.

5. Nanoparticule selon la revendication 1, pour une utilisation dans un traitement et/ou un diagnostic.

6. Nanoparticule selon la revendication 5, pour une utilisation dans le traitement et/ou le diagnostic d'un cancer chez l'homme ou un animal.

7. Nanoparticule selon la revendication 6, pour une utilisation dans la bio-imagerie, la détection de tumeurs et/ou la thermo-destruction.

8. Nanoparticule selon l'une quelconque des revendications 1 à 7 ayant un diamètre compris entre 2 et 20 nanomètres.

9. Procédé de production d'une nanoparticule selon l'une quelconque des revendications 1 à 8, le procédé comprenant la réalisation de la conversion d'un matériau précurseur de noyau de nanoparticule en or, le dépôt de la couche de tellurure de zinc sur le noyau d'or et le coiffage du tellurure de zinc avec de la cystéine.

10. Procédé selon la revendication 9, dans lequel le matériau précurseur de noyau de nanoparticule est le chlorure d'or.

11. Procédé de production d'un nanomatériau d'or-tellurure de zinc coiffé avec de la cystéine, le procédé comprenant les étapes consistant à :
a. réduire un sel d'or ;
b. ajouter du telluirium réduit, un sel de zinc et de la cystéine pour former une solution de points quantiques de tellurure de zinc coiffés avec de la cystéine ; et
c. ajouter le sel d'or réduit à la solution de points quantiques de tellurure de zinc coiffés avec une cystéine préparés pour produire une solution de nanoparticules noyau/enveloppe d'or-tellurure de zinc coiffées avec de la cystéine.

12. Procédé selon la revendication 13, dans lequel le sel d'or est le chlorure d'or.

13. Système d'administration de médicament à l'échelle nanométrique comprenant une nanoparticule noyau/enveloppe d'or-tellurure de zinc coiffée avec de la cystéine.
